# FASCICULE DE BREVET EUROPEEN

(11) **EP 2 185 565 B1**
(45) Date de publication et mention de la délivrance du brevet: **13.08.2014**
(21) Numéro de dépôt: 08805961.3
(22) Date de dépôt: 09.06.2008
(51) Int. Cl.: C07D 495/04, G01N 33/58, C07D 333/24, C07D 233/32, C07C 245/14, C12Q 1/68

(54) **Réactifs de marquage portant des fonctions diazo et nitro, procédés de synthèse de tels reactifs et procédés de détection de molécules biologiques**
Markierung von Diazo- und Nitrofunktionen übernehmenden Reagenzien, Verfahren zur Synthese solcher Reagenzien und Verfahren zur Detektion biologischer Moleküle
Marking reagents bearing diazo and nitro functions, methods for the synthesis of such reagents and methods for detecting biological molecules

(30) Priorité: 11.06.2007 FR 0755639
(43) Date de publication de la demande: 19.05.2010
(73) Titulaire: BIOMERIEUX, 69280 Marcy-L'Etoile (FR); CNRS, 75794 Paris Cedex 16 (FR); Université de Strasbourg, 67070 Strasbourg (FR)
(72) Inventeur: LAURENT, Alain, 38100 Grenoble (FR); LAAYOUN, Ali, 38260 La Frette (FR); KOTERA, Mitsuharu, F-67000 Strasbourg (FR)
(74) Mandataire: Richaud, Fabien
(86) Numéro de dépôt international: PCT/FR2008/051026
(87) Numéro de publication internationale: WO 2009/001017

(56) Documents cités:
- WO-A-02/090319
- WO-A-2005/092910
- SHIGA M ET AL: "SYNTHESIS OF A NOVEL BIOTIN DERIVATIVE THAT BEARS A DIAZO GROUP AS THE REACTIVE SITE" août 1993 (1993-08), ANALYTICAL SCIENCES, JAPAN SOCIETY FOR ANALYTICAL CHEMISTRY, TOKYO,, JP, PAGE(S) 553-556 , XP008005959 ISSN: 0910-6340 le document en entier
- BAYER E A ET AL: "THE USE OF THE AVIDIN-BIOTIN COMPLEX AS A TOOL IN MOLECULAR BIOLOGY" METHODS OF BIOCHEMICAL ANALYSIS, NEW YORK, NY, US, vol. 26, 1980, pages 1-45, XP000764016 ISSN: 0076-6941

## Description

La présente invention concerne de nouveaux réactifs de marquage de molécules biologiques, un procédé de synthèse desdits marqueurs ainsi que des applications pour le marquage de molécules biologiques en particulier dans le domaine du diagnostic utilisant l'analyse des acides nucléiques.

L'état de la technique montre que de nombreuses méthodes existent pour marquer des nucléotides, des oligonucléotides ou des acides nucléiques.

Une première méthode consiste à fixer le marqueur sur la base, que celle-ci soit naturelle ou modifiée. Une deuxième méthode propose de fixer le marqueur sur le sucre, là encore qu'il soit naturel ou modifié. Une troisième méthode a pour objet la fixation du marqueur sur le phosphate.

Le marquage sur la base a été notamment utilisé dans l'approche de marquage des acides nucléiques par incorporation de nucléotides directement marqués.

Le marquage sur le sucre est souvent utilisé dans le cas des sondes nucléiques préparées par synthèse chimique.

Le marquage sur le phosphate a été aussi utilisé pour introduire des bras fonctionnalisés et des marqueurs lors de la synthèse chimique des oligonucléotides.

En fait l'homme du métier, qui doit effectuer un marquage d'un nucléotide ou d'un analogue de nucléotide ou d'un acide nucléique, est enclin à effectuer cette fixation sur la base ou sur le sucre qui lui offrent plus de commodité et d'alternatives. C'est d'ailleurs ce qui ressort de l'étude de nombreux documents, tels que EP-A-0.329.198, EP-A-0.302.175, EP-A-0.097.373, EP-A-0.063.879, US-A-5,449,767, US-A-5,328,824, WO-A-93/16094, DE-A-3.910.151, EP-A-0.567.841 pour la base ou EP-A-0.286.898 pour le sucre.

La fixation du marqueur sur le phosphate est une technique plus complexe que la technique consistant à fonctionnaliser la base ou le sucre et a été bien moins utilisée notamment à cause de la faible réactivité du phosphate (voir par exemple Jencks W.P. et al J. Amer. Chem Soc., 82, 1778-1785, 1960). De même dans la revue de O'Donnel et Mc Laughlin (« Reporter groups for the analysis of nucleic acid structure », p 216-243, dans « Bioorganic Chemistry : Nucleic Acids », Ed Hecht S.M., Oxford University Press, 1996) portant sur les méthodes d'introduction de sondes dans les fragments d'oligonucléotides, l'alkylation efficace du phosphodiester internucléotidique est considérée comme étant impossible.

La demande de brevet WO-A-99/65926 décrit un procédé de marquage d'un acide ribonucléique (ARN) de synthèse ou naturel qui consiste à fragmenter l'ARN et à marquer au niveau du phosphate terminal. Ce document décrit un certain nombre de fonctions pouvant être utilisées pour le marquage en liaison avec la fragmentation comme les fonctions hydroxyle, amine, hydrazine, alcoxyamine, halogénure d'alkyle, halogénure d'alkyle de type benzylique et en particulier le dérivé 5-(bromométhyl)fluorescéine. Ces fonctions permettent de marquer les acides nucléiques, mais il faut associer une étape de fragmentation pour avoir un marquage efficace car ce marquage se produit sur le phosphate libéré lors de la fragmentation. De plus, il faut ajouter un excès important de réactif de marquage par rapport à l'ARN pour obtenir un marquage efficace ce qui induit des problèmes de bruit de fond générés par le marqueur en excès. Enfin, cette méthode ne fonctionne pas efficacement sur de l'ADN double brin.

Il existe donc un besoin pour de nouveaux réactifs qui soient efficaces du point de vue du rendement de marquage, qui soient spécifiques au niveau de la position de marquage et en particulier qui n'affectent pas les propriétés d'hybridation des bases impliquées dans la formation de la double hélice, par l'intermédiaire des liaisons hydrogènes, qui soient utilisables à la fois pour l'ADN et l'ARN, et enfin qui permettent de marquer indifféremment des nucléotides, des oligonucléotides, des acides nucléiques naturels ou préparés par transcription, par transcription inversée ou par amplification enzymatique.

La Demanderesse a déjà proposé de tels nouveaux marqueurs qui répondent aux conditions précitées et qui utilisent la fonction diazométhyle comme fonction réactive pour le marquage. C'est par exemple le cas dans les demandes de brevet WO-A-02/090319, WO-A-02/090584 et WO-A-2005/092910.

Ainsi la fonction diazométhyle (de formule -C(N₂)-) a déjà été utilisée pour l'alkylation des groupements phosphates, mais un certain nombre de problèmes se pose. D'une part, les réactifs incorporant au moins une fonction diazo en général sont instables par eux-mêmes, ce qui pose des problèmes pour l'utilisation de ces réactifs dans un kit de marquage, ce qui est rédhibitoire si le produit marqué a pour fonction de mettre en évidence la présence d'une molécule cible biologique dans un échantillon quelconque.

Enfin les réactifs portant la fonction diazométhyle et associés à certains marqueurs, tels que la biotine, sont peu solubles dans l'eau, ce qui conduit à utiliser des solvants organiques qui sont miscibles à l'eau pour le couplage avec des molécules biologiques, qui ne sont solubles que dans l'eau ou dans des tampons aqueux. Mais ces solvants, s'ils sont utilisés en concentration trop importante dans la réaction de marquage, risquent de provoquer la précipitation des biomolécules. Il convient donc de pouvoir disposer de réactifs de marquage suffisamment solubles dans les milieux aqueux.

Les réactifs de marquage préconisés par les documents WO-A-02/090319, WO-A-02/090584 (molécules de première génération) et WO-A-2005/092910 (molécules de deuxième génération) ci-dessus mentionnés résolvent aussi ces problèmes techniques. Le lecteur est invité à se référer à ces documents pour toute explication complémentaire qui viendrait, par omission involontaire, à manquer dans le texte exposant la présente invention.

La présente invention est une amélioration conséquente des molécules existantes. En effet les molécules de première et de deuxième générations ont l'inconvénient d'être instables chimiquement, même si ce point a déjà été amélioré par rapport à ce qui existait avant. Ainsi le marquage reste performant car les résultats obtenus sont très bons même sur plus d'un an. De plus leur synthèse reste relativement complexe. Les molécules de troisième génération sont beaucoup plus stables et plus faciles à synthétiser ce qui présente des avantages considérables en terme de date de péremption des kits contenant ces molécules et d'industrialisation des synthèses.

Les molécules de première et de deuxième générations sont stables fonctionnellement sur une année si elles sont conservées à basse température en solvant organique anhydre. Les molécules de troisième génération sont beaucoup plus stables fonctionnellement et chimiquement que ce soit en milieu liquide ou sec. Elles sont donc manipulables en milieu aqueux après avoir été conservées à sec, par séchage ou lyophilisation par exemple, sur une durée beaucoup plus longue (entre 10 et 100 fois plus), ce qui n'est pas le cas des molécules de première ou de deuxième génération qui ne résistent pas à la lyophilisation.

Cette valorisation industrielle des molécules de troisième génération est particulièrement importante dans des dispositifs intégrés ou des microsystèmes, où la chimie embarquée se doit d'être performante et robuste sans devoir incriminer en cas de problème la stabilité de certains réactifs.

Toutefois même si ces molécules et procédés de marquage sont particulièrement efficaces, la Demanderesse a réussi à trouver de nouvelles molécules et de nouveaux procédés qui améliorent encore l'efficacité du marquage. La présente invention apporte une solution afin de répondre au problème de la stabilité des molécules de première et de deuxième générations. Ainsi deux nouvelles fonctionnalités moléculaires ont été associées pour créer ces nouveaux réactifs. Elles sont définies de la manière suivante :
- la fonction diazométhyle possède en alpha un groupement aromatique, lui-même substitué une ou plusieurs fois par un groupement nitro (NO₂) en position *meta, para* ou *ortho.*
- la fonction diazo possède en alpha' le groupement permettant la détection. Ce groupement peut être la
biotine ou tout autre groupement permettant la détection.

Ce second point est une caractéristique tout à fait originale par rapport aux molécules de première et de deuxième générations car auparavant le groupement, permettant la détection, était toujours lié à la partie aromatique, la position alpha' n'étant occupée au mieux que par au moins un substituant non fonctionnel.

Il est à noter que même si la position *ortho,* de la fonction diazo par rapport au groupement nitro tous deux portés par le cycle, améliore les performances de stabilité des molécules selon l'invention, les positions *meta* et *para* conduisent à des composés diazo plus stables et sont les positions de substitution utilisées préférentiellement. Toutefois il est tout à fait possible d'améliorer encore la stabilité des molécules dont la fonction diazo est en position *ortho* du groupement nitro, en ajoutant des moyens de stabilisation, le problème étant alors que la synthèse est plus complexe.

Les définitions des « structure multimérique », « marqueur détectable », « systèmes indirects », « fluorophores » et autres marqueurs d'intérêt, « conjugaison », « molécule biologique », « acide nucléique », « technique d'amplification enzymatique », « solution sensiblement aqueuse », « solution homogène », « support solide », « étape de purification » sont données dans la demande de brevet WO-A-2005/092910, le lecteur est invité à s'y référer en cas de besoin.

De même, les techniques de :
- la chimie de greffage, et
- l'introduction de phosphate à l'extrémité 3' ou 5' des acides nucléiques,
sont également décrites dans cette demande de brevet ci-dessus et le lecteur peut retrouver dans ce document toutes informations nécessaires à la complète compréhension de la présente invention.

De plus la fonction diazométhyle portée par ces molécules de troisième génération, à l'instar des molécules de première et deuxième générations, permet de greffer de manière covalente les acides nucléiques sur le support. Le greffage est simple, la liaison est stable, par rapport à l'adsorption notamment, et permet de réaliser un couplage de l'acide nucléique sur le support solide, ce qui facilite d'autant les étapes d'hybridation ultérieures en diminuant l'encombrement stérique.

Cette nouvelle famille de molécules est appelée Diazo Cétone Biotine (DCB) et est représentée par le réactif de marquage relativement stable à la température de formule (A) ci-dessous :

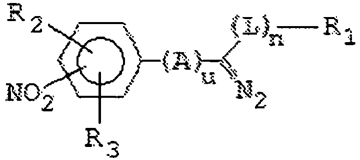

Cette molécule de troisième génération DCB comporte un bras espaceur L, appelé linker, et un marqueur R₁ qui pourrait être constitué par un groupement détectable, tel que la biotine, un haptène, un fluorophore, un groupement fluorescent, un groupement luminescent, etc.

L est un bras de liaison comportant un enchaînement linéaire d'au moins deux liaisons covalentes et n un nombre entier égal à 1. Avantageusement et quel que soit le mode de réalisation ou variante précédemment évoqué(e) du réactif, L comprend un motif - (O-CH₂-CH₂)-, répété de 1 à 20 fois, préférentiellement de 1 à 10 fois, et encore plus préférentiellement de 2 à 5 fois.

A est un bras de liaison comportant au moins une double liaison covalente permettant la conjugaison de la fonction diazo avec le cycle aromatique et u est un nombre entier compris entre 0 et 2, préférentiellement de 0 ou 1.

Dans un mode particulier de réalisation, A est un bras de liaison comportant au moins une double liaison de type éthylénique permettant la conjugaison de la fonction diazométhyle avec le cycle aromatique. Le bras de liaison A a pour fonction d'éloigner la fonction diazométhyle du cyle pour diminuer l'encombrement stérique tout en conservant la stabilité de la fonction diazométhyle. Par « conjugaison », on entend la délocalisation électronique du cycle aromatique le long de la chaîne carbonée du bras de liaison A. A titre d'exemple, le bras A peut avoir la structure suivante : 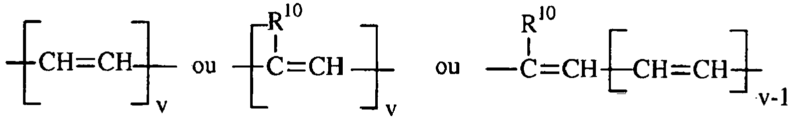 dans laquelle :
- v est un nombre entier compris entre 1 et 10, de préférence v est égal à 1 ou 2, et
- R¹⁰ est H ou un groupement alkyle, de préférence R¹⁰ est H, méthyle ou éthyle.

Les radicaux R₂ et R₃ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Selon un deuxième mode de réalisation, le réactif de marquage est de formule (C) telle que décrite ci-dessous : 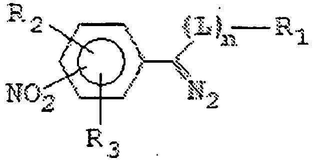 dans laquelle :
- R₁ représente un marqueur détectable ou au moins deux marqueurs détectables reliés entre eux par au moins une structure multimérique,
- R₂ et R₃ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle,
- L est un bras de liaison comportant un enchaînement linéaire d'au moins deux liaisons covalentes, et
- n un nombre entier égal à 1.

Selon un troisième mode de réalisation, le réactif de marquage est de formule (E), comme décrit ci-dessous :

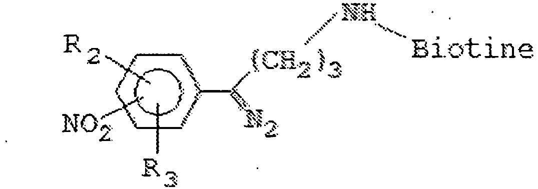

Quel que soit le mode réalisation, le réactif se caractérise notamment par le fait le groupement nitro est en position *meta* ou *para.*

Dans un mode intéressant de réalisation, le groupement R₁ est consituté par un résidu D-Biotine de formule (F) :

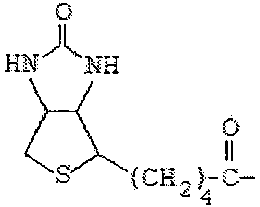

L'invention concerne également un procédé de synthèse d'un réactif de marquage tel que décrit ci-dessus, ce procédé comprend les étapes suivantes :
a) un dérivé d'acide carboxylique est mis en réaction avec l'énolate d'une lactone (réaction de type Claisen) pour former un précurseur cyclique,
b) lequel précurseur cyclique est ensuite ouvert avec un acide halogéné pour former une cétone aromatique halogénée,
c) la fonction carbonyle de la cétone aromatique halogénée est protégée par un groupement protecteur pour former un précurseur protégé,
d) lequel précurseur protégé est soumis à une réaction d'amination (de type Gabriel) pour former un précurseur aminé,
e) lequel précurseur aminé est déprotégé pour libérer la fonction amine, ladite fonction amine étant mise en réaction avec un marqueur détectable dont la fonction carboxylique est activée pour former un précurseur comportant un marqueur détectable,
f) le précurseur marqué est soumis à une réaction de déprotection de la fonction carbonyle pour former un précurseur marqué et carbonylé, enfin
g) le précurseur marqué et carbonylé est transformé en un réactif de marquage, tel que décrit ci-dessus, par une transformation de la fonction carbonyle en une fonction diazo (du type Bamworth Stevens).

Dans un mode de réalisation, le procédé pour le marquage d'une molécule biologique, en particulier un acide nucléique, comprend la mise en contact en solution homogène, dans un tampon sensiblement aqueux, d'une molécule biologique et d'un réactif, tel que décrit précédemment.

L'invention concerne également une molécule biologique marquée susceptible d'être obtenue par le procédé, précédemment décrit.

L'invention concerne aussi un procédé de marquage et de fragmentation d'un acide nucléique simple ou double brin comprenant les étapes suivantes :
- fragmenter l'acide nucléique,
- attacher un marqueur sur au moins un des fragments par l'intermédiaire d'un réactif de marquage choisi parmi les réactifs, tel que décrit précédemment,
ledit réactif se couplant de manière covalente et majoritaire sur au moins un phosphate dudit fragment.

Selon un mode de réalisation, le procédé est caractérisé par le fait que la fragmentation et le marquage sont effectués en deux étapes.

Selon un autre mode de réalisation, le procédé est caractérisé par le fait que la fragmentation et le marquage sont effectués en une étape.

Quel que soit le mode de réalisation, le procédé se caractérise par le fait que le marquage s'effectue en solution homogène sensiblement aqueuse.

Quel que soit le mode de réalisation, le procédé est caractérisé par le fait que la fragmentation s'effectue par voie enzymatique, physique ou chimique.

L'invention concerne également un acide nucléique marqué susceptible d'être obtenu par le procédé, selon l'un quelconque des modes de réalisation ci-dessus décrit.

L'invention concerne encore un kit de détection d'un acide nucléique cible comprenant un acide nucléique marqué, tel que celui-ci ci-dessus exposé.

L'invention concerne également un support solide sur lequel est fixé un réactif, tel que décrit précédemment.

L'invention concerne également un procédé de capture d'acides nucléiques comprenant les étapes suivantes :
- on dispose d'un support solide sur lequel est fixé directement ou indirectement au moins une molécule biologique ou un acide nucléique, la molécule biologique ou l'acide nucléique comprenant une fonction diazométhyle,
- on met en contact un échantillon biologique susceptible de contenir des acides nucléiques libres, et
- on lave le support solide où la (ou les) molécule (s) sont fixée(s) de manière covalente au moins à un acide nucléique.

Les exemples et figures ci-joints représentent des modes particuliers de réalisation.
La figure 1 représente un schéma réactionnel montrant le marquage d'un acide nucléique simple brin, qu'il soit sous forme d'ARN ou d'ADN par une molécule selon l'invention.
La figure 2 représente un schéma réactionnel du procédé de synthèse d'une molécule de *meta* Nitro DCB.
La figure 3 représente un schéma réactionnel du procédé de synthèse d'une molécule de *para* Nitro DCB.
La figure 4 représente la stabilité comparée en milieu liquide des DCB par rapport à une molécule de [Bio-EG3]2-PDAM de deuxième génération (ci-après appelée BBP) et décrite dans la demande WO-A-02090319.
La figure 5 représente la stabilité comparée sous forme sèche des DCB par rapport à une BBP.
La figure 6 représente les résultats de marquage d'un amplicon ARN après purification à l'aide des marqueurs BBP, *meta* Nitro DCB et *para* Nitro DCB, chacun des marqueurs étant à une concentration de 45 mM.
La figure 7 représente les résultats de marquage d'un amplicon ARN sans purification à l'aide des marqueurs BBP, *meta* Nitro DCB et *para* Nitro DCB, chacun des marqueurs étant à une concentration de 2 mM et en présence 3 mM HCL.
La figure 8 représente les résultats de marquage en fonction du temps d'un amplicon ARN à l'aide des marqueurs *meta* Nitro DCB et *para* Nitro DCB, chacun des marqueurs étant à une concentration de 3 mM et en présence de 3 mM HCL.
La figure 9 représente la comparaison de l'efficacité de marquage d'un amplicon ARN en fonction de la technique employée, soit ULS (Universal Labelling System) de Kreatech ou selon la présente invention.
La figure 10 est un récapitulatif des molécules utilisées dans la présente demande d'invention.

Dans les exemples décrits ci-après, les abréviations suivantes seront utilisées :
- Ar : aromatique,
- s : singulet,
- d : doublet,
- t : triplet,
- qu :quintuplet,
- m : massif,
- M : multiplet,
- HPLC : chromatographie liquide haute pression,
- CCM : chromatographie sur couche mince,
- RMN : résonance magnétique nucléaire,
- Rdt : rendement,
- Rf ou TR : temps de rétention,
- DMSO-d6 : diméthylsulfoxide deutéré,
- DMCF : diméthyl cyclohexylamonium formiate,
- CDCl₃ : Chloroforme deutéré, et
- DMF : diméthylformamide
- DCM : Dichlorométhane
- MeOH : Méthanol
- ACN : Acétonitrile
- Eau MilliQ : Eau ultrapure de type Millipore
- DMAC : Diméthyl amino cinnamaldehyde

### Conditions générales pour la synthèse et l'analyse des composés DCB :

Ces conditions sont applicables aux exemples 1, 2, 3 et 4 qui suivent.

Conditions HPLC (Chaîne HPLC WATERS Alliance 2795, Détecteur à barette de diodes PDA 996, logiciel Empower version 2 et colonne WATERS XTerra MS C18 4,6 x 30 2,5 µM):

### 1°) Méthode basique :

Eluant A : eau milliQ
Eluant B : ACN
Eluant C : ammoniaque à 500 mM pH 12
Soit un gradient linéaire de 1 à 64% d'acétonitrile (5 mM constants d'ammoniaque à pH=9) en 20 min.

### 2°) Méthode DMCF (Diméthyl Cyclohexylamonium Formiate) :

Eluant A : eau milliQ
Eluant B : ACN
Eluant D : DMCF à 500 mM pH=7
Soit un gradient linéaire de 1 à 64% d'acétonitrile à 5 mM de DMCF à pH=7 en 20 min.

La solution de DMCF à 500 mmol/L est préparée à partir de 37 mL de diméthylcyclohexylamine, de 9,4 mL d'acide formique pur et de 200 mL d'eau. On ajuste le pH à 7 et on complète à 500 mL avec de l'eau.

### 3°) Conditions générales de la synthèse :

Les analyses par chromatographie sur couche mince ont été réalisées sur des plaques de silice ALUGRAM^{®} MACHEREY-NAGEL SIL G/UV₂₅₄ 4 x 8 cm avec une détection UV à 254 nm ou au DMAC pour les produits biotinylés.

La purification des produits a été effectuée par chromatographie sur gel de silice Silica gel 60 FLUKA (40-63 µm). Les conditions de séparation par chromatographie « flash » (poussée sous argon) respectent strictement les conditions décrites par Clark Still *et al.* (Clark Still, W. ; Kahn, M. ; Mitra, A. J. Org. Chem. 1978, 43, 2923-2925) soit une hauteur fixe de silice de 15 cm poussée à un débit de 5 cm/min, le diamètre de la colonne dépendant de la quantité et des Rf des produits à purifier.

Les spectres RMN ont été enregistrés sur un spectromètre Brüker 200 MHz. Les déplacements chimiques (δ) sont donnés en ppm par rapport au pic du solvant pris comme référence interne (CDCl₃ : 7,24 ppm ; DMSO-d6 : 2,49 ppm ; D₂O : 4,80 ppm à 25°C). Les spectres sont décrits avec les abréviations : s = singulet, d = doublet, t = triplet, q = quadruplet, qu = quintuplet, m = massif, M = multiplet.

Les spectres de masse (SM) ont été obtenus avec un appareil LCQ-ion trap (Thermofinnigan, San Jose, CA, USA) par des méthodes d'ionisation Electrospray en mode positif par infusion à travers un tube de silice 2 à 10 µL/min. Les principaux solvants utilisés sont le DCM et le MeOH.

### Exemple 1 : Synthèse de la meta Nitro DCB :

### Objectif :

Démontrer la faisabilité de la synthèse d'une molécule DCB : la *meta* Nitro DCB.

### Mode Opératoire :

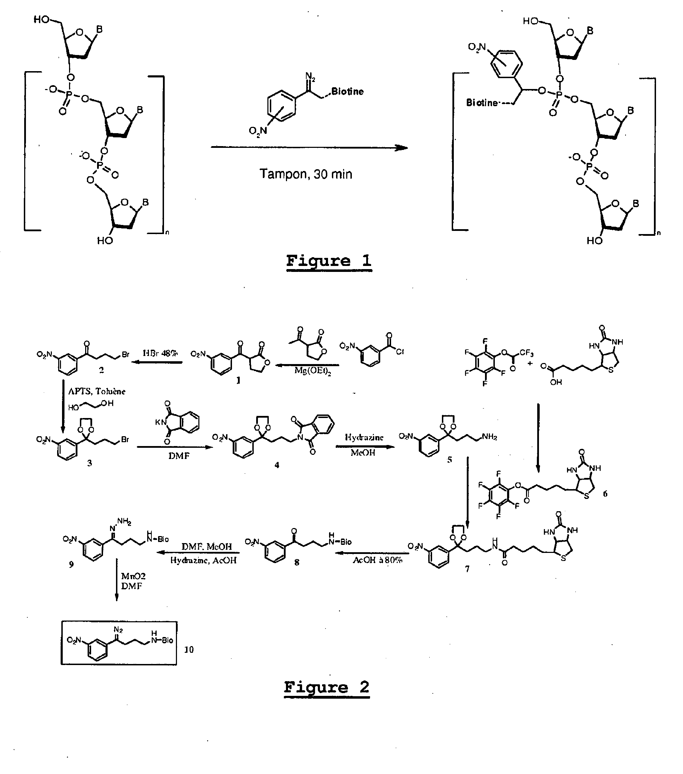

La synthèse proprement dite est présentée en figure 2. Elle débute avec une molécule de *meta* Nitro Chlorure de benzyle (3-nitrobenzoyl chlorure), qui est commercialement disponible (Aldrich, Saint Quentin Fallavier, France).

### Molécule 1

Le magnésium éthoxide (5,41 g ; 0,0473 mol) est mis en suspension dans le chlorobenzène (33 mL) suivit de l'α-acétyl-γ-butyrolactone (5,29 mL ; 0,0492 mol), l'ensemble est mis sous argon, agité et chauffé à 75°C pendant 3 h. Après avoir refroidi le milieu réactionnel à température ambiante et ajouté le 3-nitrobenzoyl chlorure (10 g ; 0,0539 mol) en solution dans du chlorobenzène (16,4 mL) on rechauffe l'ensemble à 40°C pendant 2 heures.

Le mélange est refroidi à 10°C avant d'être hydrolysé avec 25 mL d'acide sulfurique 1 M, on laisse décanter et on récupère la phase organique (phase supérieure) pour la laver avec une solution à 5% d'hydrogénocarbonate de sodium. La phase organique est ensuite évaporée à sec sous pression réduite pour finalement être purifiée sur une colonne à gel de silice de 7 cm de diamètre et de 10,5 cm de hauteur, avec une vitesse d'élution de 5 cm/min. Dans un premier temps on passe comme éluant un mélange 70/30 de cyclohexane/acétate d'éthyle suivi d'un mélange 50/50. Les fractions contenant le produit attendu sont rassemblées et évaporées à sec pour obtenir le produit 1 (8,17 g ; Rdt=73,5%), qui est directement engagé dans la réaction suivante.
Eluant CCM : dichlorométhane/méthanol : 95/5
HPLC : Méthode DMCF ; TR=7,7 min

### Molécule 2

La molécule 1 (5,50 g ; 0,0234 mol) est mis en suspension dans une solution à 48% d'acide bromhydrique aqueux (33 mL ; 6 v) que l'on porte au reflux pendant 2 h. Après avoir refroidi la solution à température ambiante on extrait la phase aqueuse avec 3 fois 30 mL de dichlorométhane. La phase organique est contre extraite à l'hydrogénocarbonate de sodium à 5% puis séchée au sulfate de sodium filtrée et évaporée. On obtient le produit 2 (6,06 g ; Rdt=95,8%)
Eluant CCM : dichlorométhane/méthanol : 95/5
HPLC : Méthode DMCF ou basique ; TR=13,6 min
RMN ¹H (200 MHz, DMSO) : 2,416 ppm (Qu, 2H, CH₂-CH₂Br), 3,426 ppm (t, 2H, CH₂Br), 3,279 ppm (t, 2H, CH₂-CO), 7,738 ppm (t, 1H, Ar), 8,328 (d, 1H, Ar), 8,457 (d, 1H, Ar), 8,837 (s, 1H, Ar).

### Molécule 3

Dans un ballon monté avec un Dean Stark, on mélange le composé 2 (4,00 g ; 14,7 mmol), l'éthylène glycol (2.87 mL ; 51,4 mmol), l'acide paratoluènesulfonique (139,6 mg ; 7,35 mmol) et le toluène (50 mL ; 12,5 v) puis on porte au reflux pendant 7h au total (5h30 à la consigne de 130°C puis 1h30 à 150°C). La réaction est totale, on évapore le toluène sous pression réduite, on reprend par 70 mL d'acétate d'éthyle et on lave avec deux fois 50 mL d'une solution de l'hydrogénocarbonate de sodium à 250 mmol/L puis avec deux fois 50 mL d'eau, la phase organique est séchée sur du sulfate de sodium, filtrée et évaporée à sec avant d'être purifiée par chromatographie sur gel de silice (Φ=7cm, h=7cm, d=5cm/min) avec comme éluant : cyclohexane/acétate d'éthyle : 85/15. Après cette colonne on obtient le composé 3 (3,70 g ; Rdt=79,6%)
Eluant CCM : cyclohexane/acétate d'éthyle : 85/15
HPLC : Méthode basique ; TR=15,3 min
RMN ¹H (200 MHz, DMSO) : 1,90-2,10 ppm (m, 4H, CH₂-CH₂-CH₂Br), 3,425 ppm (t, 2H, CH₂Br), 3,798 ppm (t, 2H, CH₂-O), 4,081 ppm (t, 2H, CH₂-O), 7,561 ppm (t, 1H, Ar), 7,75-7,85 ppm (m, 1H, Ar), 8,10-8,25 (m, 1H, Ar), 8,30-8,35 (m, 1H, Ar)

### Molécule 4

Le produit 3 (3,70 g ; 11,7 mmol) est dissout dans le DMF (40 mL, 0,3 M) avant d'ajouter le phtalimide de potassium (3,25 g ; 17,5 mmol) en une fois et de chauffer 15 min à 155°C. On évapore le DMF sous pression réduite, puis on reprend le résidu d'évaporation avec 60 mL de dichlorométhane, on lave avec 60 mL d'une solution de soude à 0,1 mol/L, on contre-extrait la phase aqueuse avec 30 mL de dichlorométhane, on assemble les deux phases organiques pour les sécher sur un lit de sulfate de sodium avant de filtrer et d'évaporer à sec la solution en distillant le solvant. On reprend le résidu avec de l'éthanol (55 mL ; 12 v) et on porte au reflux 20 min (la solubilisation n'est pas totale) avant de refroidir à 10°C, le produit précipite, on l'essore sur un verre frité, le produit 4 est lavé avec deux fois 9,2 mL d'éthanol puis séché sous vide (3,99 g ; Rdt=89,1%)
HPLC : Méthode basique ; TR=14,9 min
RMN ¹H (200 MHz, CDCl₃) : 1,604-2,005 ppm (m, 4H, CH₂-CH₂-CH₂-N), 3,677-3,783 ppm (m, 4H, O-CH₂-CH₂-O), 4,019 ppm (t, 2H, CH₂-N), 7,500 (t, 1H, Ar), 7,600-8,000 (m, 4H, Phtalimide), 8, 150 (d, 1H, Ar), 8,300 (s, 1H, Ar)

### Molécule 5

La substance 4 (3, 987 g ; 10,4 mmol) est mis en suspension dans le méthanol (210 mL ; 53 v) avant d'ajouter à température ambiante l'hydrazine (15,2 mL ; 313 mmol). La réaction est terminée après 20 min. On évapore à sec le milieu réactionnel puis on le reprend avec 100 mL d'acétate d'éthyle et on lave avec 100 mL d'eau, on extrait la phase aqueuse obtenue avec deux fois 100 mL d'acétate d'éthyle suivit de trois fois 100 mL de dichlorométhane. Toutes les phases organiques sont assemblées, évaporées sous pression réduite et déposées sur une colonne de gel de silice (Φ=5 cm, h=15 cm, d=5 cm/min) avec comme éluant dichlorométhane/méthanol/ammoniaque : 90/10/1

Au final on obtient le composé 5 (2,11 g ; Rdt=80,1%).
Eluant CCM : dichlorométhane/méthanol/ammoniaque : 90/10/3 HPLC : Méthode basique ; TR=4,8 min
RMN ¹H (200 MHz, DMSO) : 1,464-1,666 ppm (m, 4H, CH₂-CH₂-NH₂), 1,904-1,985 ppm (M, 2H, -CH₂-CH₂-CH₂-NH₂); 2,689 ppm (t, 2H, CH₂-NH₂), 3,753-4,115 ppm (m, 4H, O-CH₂-CH₂-O), 7,540 ppm (t, 1H, Ar), 7,837 ppm (d, 1H, Ar), 8,147 ppm (d, 1H, Ar), 8,331 ppm (s, 1H, Ar)

### Molécule 6

La biotine (6,00 g ; 24,5 mmol) est mis en solution dans le DMF anhydre (60 mL ; 10 v) puis on additionne sous argon la pyridine (2,44 mL ; 30,0 mmol) suivit du pentafluorophenyl trifluoroacétate (5,258 mL ; 30,5 mmol), on chauffe 30 min à 40°C puis on laisse revenir à température ambiante pour la nuit. Après contrôle, la réaction est terminée, on évapore le DMF et on reprend avec 200 mL de dichlorométhane, le produit reste en suspension, on essore, on rince le gâteau avec trois fois 5 mL de dichlorométhane, puis on sèche à l'étuve sous vide à 25°C. On obtient un produit très propre en CCM (6,18 g ; 61,3%).
Eluant CCM : dichlorométhane/méthanol : 90/10
HPLC : Méthode basique ; TR=10,0min
RMN ¹H (200 MHz, DMSO) : 1,2-1,8 ppm (m,6H), 2,3-3,4 ppm (m, 5H), 4,0-4,4 ppm (m, 2H), 6,1-6,6 ppm (m, 2H)

### Molécule 7

Le réactif 5 (500 mg ; 1,98 mmol) est dissout dans un mélange de DMF (5 mL ; 10 v) à 60°C et de triéthylamine (2,22 mL ; 15,8 mmol), parallèlement le composé 6 est aussi dissout dans 4,9 mL de DMF à 60°C. On coule la solution de 6 sur la solution de 5, on laisse agiter une nuit à température ambiante. On évapore à sec sans traitement supplémentaire.

### Molécule 8

La substance 7 (948 mg ; 1,98 mmol) est mise en réaction avec de l'acide acétique à 80% (5 mL ; 5,3 v) au reflux pendant 16 heures (note : on peut utiliser de l'acide chlorhydrique 6 M, beaucoup plus réactif). On suit la réaction par HPLC, il faut qu'elle soit totale. L'acide acétique est évaporé par distillation sous vide puis le résidu est repris par 500 mL de mélange dichlorométhane/méthanol : 90/10 et lavé avec 250 mL de solution d'hydroxyde de soude à 0,1 M. La phase organique ainsi purifiée est séchée sur du sulfate de sodium filtrée et évaporée à sec. On récupère le composé 8 (244,8 mg ; 28,4%). Le produit est utilisé brut sans traitement supplémentaire.
Eluant CCM : non utilisé, impossible de différentier le produit de départ de celui d'arrivée
HPLC, Méthode basique TR=9,9 min

### Molécule 9

Le composé 8 est repris dans un mélange de DMF (2,49 mL ; 12,3 v) et de méthanol (12,5 mL ; 51 v) on y ajoute de l'acide acétique (640 µL ; 11,1 mmol), le produit de départ n'est pas soluble. Finalement on met l'hydrazine (271 µL ; 5,57 mmol), le réactif se solubilise et la solution devient jaune. Après 5 heures d'agitation à température ambiante, on évapore tous les solvants on les co-évapore avec trois fois 10 mL d'eau. On reprend le brut de réaction dans un tube de 15 mL avec 8 mL d'eau milliQ à 5°C, le produit précipite, on agite 30 secondes puis on centrifuge le tube à 8500 tr/min pendant 3 min, on aspire le surnageant, et on recommence l'opération deux fois. Lors du dernier lavage on vérifie le pH de l'eau, elle est bien à 7, il n'y a plus de trace d'acide.
HPLC : Méthode basique ; TR=8,4 min
RMN ¹H (200 MHz, DMSO) : 1,1-1,9 ppm (m, 8H, -CH₂-), 2,085 ppm (t, 2H, CO-CH₂), 2,50-2,90 ppm (m, 4H, Biotine), 3,00-3,20 ppm (m, 3H), 3,30-3,40 ppm (m, 2H), 4,0-4,4 ppm (m, 2H, biotine), 6,30 et 6,50ppm (2s, 2H, NH biotine), 7,23 ppm (s, 2H, NH), 7,866 ppm (d, 2H, Ar), 8,155 (s, 2H, Ar)

### Molécule 10

La poudre 9 (250 mg ; 0,56 mmol) est repris dans du DMF (4,65 mL ; 18,6 v), refroidit à -5°C pour y ajouter le tetramethylguanidine (571 µL ; 4,53 mmol) suivit de tamis moléculaire 3Å (957 mg) et de l'oxyde de manganèse (3,03 g ; 45,3 mmol). On laisse agiter sous argon 40 min à -5°C puis on filtre le milieu réactionnel sur un bouchon d'1 cm de célite, le bouchon est ensuite rincé avec du méthanol jusqu'à ce que le filtrat devienne incolore. On récupère ce filtrat, on l'évapore sous pression réduite (température du bain : 35°C maximum). Le résidu d'évaporation est repris dans un tube de 15 mL par 4 mL de mélange dichlorométhane/méthanol : 90/10 puis on le lave avec 4 mL d'une solution d'hydrogénocarbonate de sodium à 0,1 M. On élimine le surnageant après centrifugation. A la phase dichlorométhane on rajoute 350 µL de méthanol et 4 mL d'hydrogénocarbonate de sodium, on mélange, on décante, on élimine la phase aqueuse, on sèche la phase organique finale avec du carbonate de sodium anhydre, on filtre et le filtrat est évaporé à sec sous pression réduite. On obtient une poudre orange (196,8 mg ; Rdt=79,3% sur les deux dernières étapes).
HPLC : Méthode basique TR=11,05 min
RMN ¹H (200 MHz, DMSO) : 1,1-1,9 ppm (m, 8H, -CH₂-), 2,058 ppm (t, 2H, CO-CH₂), 2,4-3,4 ppm (m, 7H), 3,0-3,2 ppm (m, 5H), 4,0-4,3 ppm (m, 2H, biotine), 6,33 et 6,40 ppm (2s, 2H, NH biotine), 7,36 ppm (d, 1H, Ar), 7,50-7,70 ppm (m, 2H, Ar+NH), 7, 80-7, 90 ppm (m, 3H, Ar).

### Résultats et conclusions :

Nous avons montré que la synthèse d'une troisième génération de molécules était possible. Cette synthèse est beaucoup plus simple que celles associées aux précédentes générations de molécules.

Ainsi les molécules de deuxième génération nécessitent une synthèse en onze étapes relativement complexes et laborieuses.

D'autre part, la voie de synthèse des molécules de deuxième génération est peu versatile. La nouvelle voie de synthèse faisant l'objet de l'invention est beaucoup plus versatile, c'est-à-dire que d'autres substrats aromatiques peuvent être utilisés en suivant la même voie pour augmenter la diversité des molécules finales.

### Exemple 2 : Synthèse de la para Nitro DCB :

### Objectif :

Démontrer la faisabilité de la synthèse d'une molécule DCB, la *para* Nitro DCB.

### Mode Opératoire :

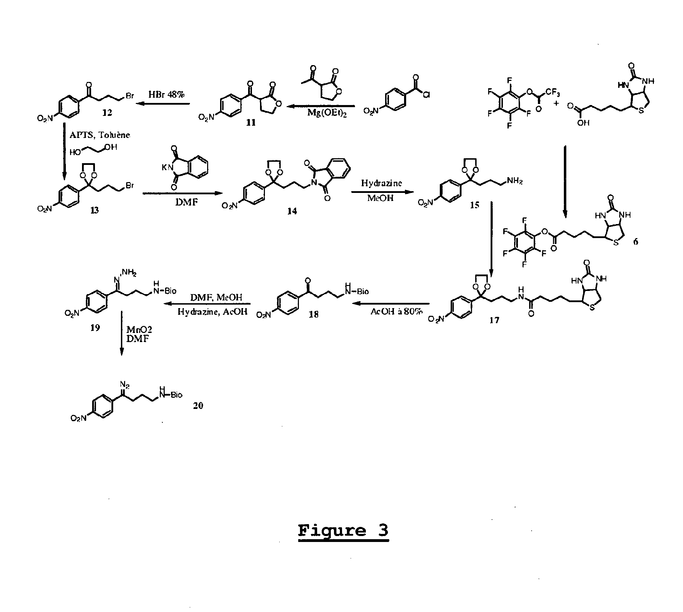

La synthèse proprement dite est présentée en Figure 3. Le produit de départ est le *para* Nitro Chlorure de benzyle (Aldrich, St Quentin Fallavier, France). Les différentes molécules synthétisées ci-après suivent exactement les modes opératoires décrits dans l'exemple 1.

### Molécule 11

Eluant CCM : dichlorométhane/méthanol : 95/5
HPLC : Méthode basique ; Tr=1,8 min
RMN ¹H (200MHz, DMSO) : 2, 40-2, 90 ppm (m, 2H, CH₂-CH₂O), 4,432 ppm (M, 2H, CH₂O), 5,153 ppm (t, 1H, C=O-CH-C=O), 8,303 ppm (d, 2H, Ar), 8,382 ppm (d, 2H, Ar)
MS : [M-H⁺]⁻ à m/z = 234, 3.

### Molécule 12

Eluant CCM, dichlorométhane/méthanol : 95/5
HPLC : Méthode basique ; TR=13,9 min
RMN ¹H (200 MHz, DMSO) : 2,189 ppm (Qu, 2H, CH₂-CH₂Br), 3,177 ppm (t, 2H, CH₂Br), 3,624 ppm (t, 2H, CH₂-CO), 8,217 ppm (d, 2H, Ar), 8,334 (d, 2H, Ar)

### Molécule 13

Eluant CCM : cyclohexane/acétate d'éthyle : 85/15
HPLC : Méthode basique ; TR=15,5 min
RMN ¹H (200 MHz, DMSO) : 1,80-2,10 ppm (m, 4H, CH₂-CH₂-CH₂Br), 3,415 ppm (t, 2H, CH₂Br), 3,767 ppm (t, 2H, CH₂-O), 4,077 ppm (t, 2H, CH₂-O), 7,669 ppm (d, 2H, Ar), 8,186 (d, 2H, Ar)

### Molécule 14

HPLC : Méthode basique ; TR=15,1 min
RMN ¹H (200 MHz, CDCl₃) : 1,50-1,65 ppm (m, 4H, CH₂-CH₂-CH2-N), 3,65-3,70 ppm (m, 4H, CH₂-O et CH₂-N), 4,00-4,10 ppm (M, 2H, CH₂-O), 7,55-7.65 (M, 2H, Ar), 7,65-7,75 (M, 2H, Phtalimide), 7,80-7, 90 ppm (M, 2H, Phtalimide) 8,192 ppm (M, 21H, Ar)

### Molécule 15

Eluant CCM : dichlorométhane/méthanol/ammoniaque : 90/10/3
HPLC : Méthode basique ; TR=5,0 min
RMN ¹H (200 MHz, DMSO) : 1,30-1,60 ppm (m, 4H, CH₂-CH₂-NH₂), 1,80-2,00 ppm (M, 2H, -CH2-CH₂-CH₂-NH₂); 2,678 ppm (t, 2H, CH₂-NH₂), 3,769 ppm (M, 2H, O-CH₂), 4,060 ppm (M, 2H, O-CH₂), 7,66 ppm (M, 2H, Ar), 8,177 ppm (M, 2H, Ar)

### Molécules 17

HPLC : Méthode basique ; TR=9,1 min
Eluant CCM : dichlorométhane/méthanol : 90/10

### Molécule 18

Eluant CCM : non utilisé, impossible de différencier le produit de départ de celui d'arrivée.
HPLC : Méthode basique ; TR=9,0 min
RMN ¹H (200 MHz, DMSO) : 1,2-1,7 ppm (m, 8H, -CH₂-), 2,093 ppm (t, 2H, CO-CH₂), 2,4-3,0 ppm (m, 2H, Biotine), 3,0-3,2 ppm (m, 5H), 4,0-4,4 ppm (m, 2H, biotine), 6,34 et 6,40 ppm (2s, 2H, NH biotine),7,70-7,90 ppm (m, 2H, Ar+NH), 8,20-8,50 ppm (m, 2H, Ar), 8,63 ppm (s, 1H, Ar)

### Molécule 19

HPLC : Méthode basique ; TR=8,2 min

### Molécule 20

HPLC, Méthode basique ; TR=10,7 min
RMN ¹H (200 MHz, DMSO) : 1,2-1,9 ppm (m, 8H, -CH₂-), 2,066 ppm (t, 2H, CO-CH₂), 2,5-3,0 ppm (m, 4H), 3,0-3,2 ppm (m, 2H), 4,00-4,40 ppm (m, 2H, biotine), 6,36 et 6,42 ppm (2s, 2H, NH biotine), 7,19 ppm (d, 2H, Ar), 7,875 ppm (t, 1H, NH), 8,133 ppm (d, 2H, Ar).

### Résultats et conclusions :

Les étapes de synthèse de la molécule de *para* Nitro DCB sont identiques à celles décrites précédemment pour la synthèse de la molécule de *meta* Nitro DCB, il suffit pour cela de commencer avec une molécule de *para* Nitro Chlorure de benzyle, dite 4-nitrobenzoyl chlorure en lieu est place de la molécule de *meta* Nitro Chlorure de benzyle, dite 3-nitrobenzoyl chlorure

### Exemple 3 : Démonstration de la stabilité de la meta ou para Nitro DCB par rapport à une molécule de BBP (bis-bio-PDAM) en milieu liquide et à température ambiante :

### Objectif :

Démontrer la stabilité en milieu liquide des molécules DCB en comparaison à une molécule de deuxième génération. Pour cela une étude de stabilité accélérée est effectuée dans des conditions extrêmes où les composés sont conservés à 125 mM dans un mélange DMSO/méthanol 96/4 à température ambiante (22°C +/- 1°C). A noter que ce sont des conditions extrêmes de conservation.

### Mode Opératoire :

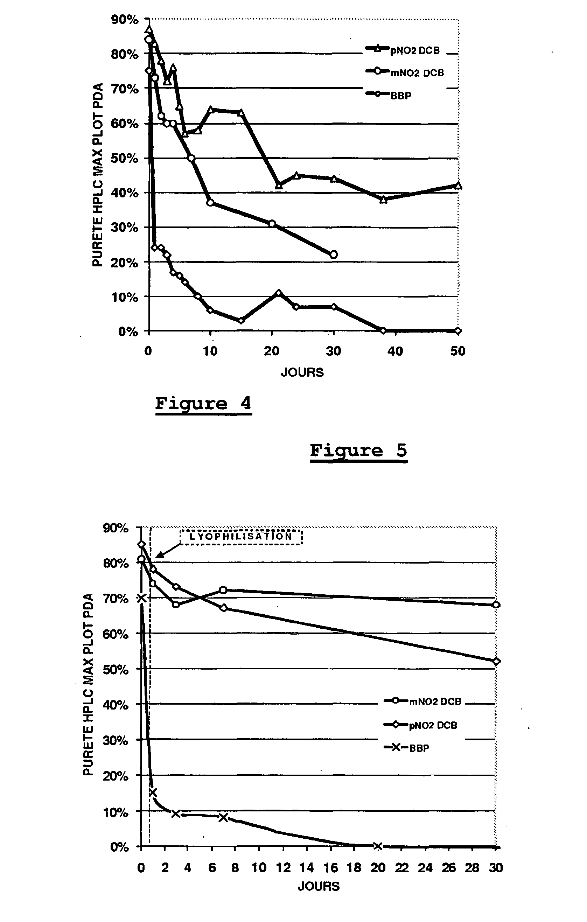

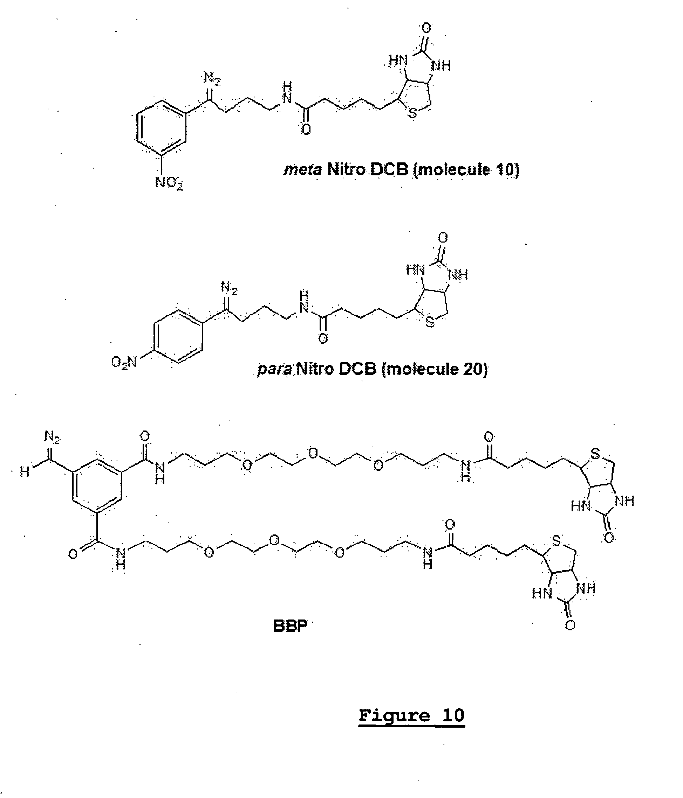

Les trois composés *meta* Nitro DCB, *para* Nitro DCB et le BBP, bien représentées sur la figure 10, sont solubilisés à 125 mM dans un mélange DMSO/méthanol 96/4 et conservés à température ambiante (22°C à plus ou moins 1°C). On injecte alors régulièrement en HPLC (méthode basique, chaîne HPLC Waters) 2 µL de ces solutions, afin de mesurer la dégradation du produit principal par intégration de l'ensemble des pics du chromatogramme (PDA Max Plot sur le logiciel Empower 2). On reporte alors l'évolution de la pureté du composé initial en fonction du temps, comme cela est bien représenté sur la figure 4.

### Résultats et conclusions :

On démontre sans ambiguité que le BBP se dégrade en quelques jours (<10% de pureté à 10 jours) alors que les DCB demeurent beaucoup plus stables (> 40% de pureté après 2 mois de conservation pour le *para* Nitro DCB). La combinaison du motif nitro aryl en alpha et du marqueur en alpha' de la fonction diazo stabilise cette dernière, à la fois par une délocalisation électronique et par un effet stérique qui rendent la fonction diazo moins succeptible à l'hydrolyse.

### Exemple 4 : Démonstration de la stabilité de la meta ou para Nitro DCB par rapport à une molécule BBP, sous forme sèche et à 4°C :

### Objectif :

Démontrer la stabilité à sec des molécules DCB en comparaison à une molécule BBP de deuxième génération.

### Mode Opératoire :

Les trois composés *meta* Nitro DCB, *para* Nitro DCB et le BBP sont solubilisés à 250 mM dans une solution de Tris HCl 10 mM pH 7,5 et de Tréhalose à 10 %. Les solutions sont lyophilisées pendant une nuit par aliquots de 50 nmol. Les produits secs sont ensuite conservés à 4°C. Régulièrement, on solubilise ces aliquots dans du méthanol et on injecte en HPLC (Waters) 15 µL de ces solutions, afin de mesurer la dégradation du produit principal par intégration de l'ensemble des pics du chromatogramme (PDA Max Plot sur le logiciel Empower). On reporte alors l'évolution de la pureté du composé initial en fonction du temps, comme cela est bien représenté sur la figure 5.

### Résultats et conclusions :

De la même manière que dans l'exemple 3 mais de façon encore plus notable on démontre que le BBP ne supporte pas la lyophilisation (>60% de dégradation pendant cette étape) alors que les DCB restent parfaitement stables et en particulier pour le *meta* Nitro DCB. Encore une fois on démontre que la combinaison du motif nitro aryl en alpha de la fonction diazo et du marqueur en alpha' stabilise considérablement cette fonction en la rendant moins susceptible à l'hydrolyse.

### Exemple 5 : Marquage d'acides nucléiques par la meta ou para Nitro DCB par rapport à une molécule de BBP avec purification intermédiaire :

### Objectif :

Démontrer l'efficacité du marquage des acides nucléiques par des molécules DCB en comparaison à une molécule de deuxième génération (BBP).

Pour cela des amplicons d'ARN, fragments de la séquence de *Mycobacterium tuberculosis* de 174 bases, issus d'une réaction d'amplification (NASBA, kit NucliSens Basic Kit de bioMérieux B.V.- Boxtel - Pays-Bas) sont marqués à la biotine par réaction avec des DCB. Les produits de la réaction de marquage sont détectés par hybridation sur une puce à ADN d'Affymetrix (Custom DNA Chip Combo Myco décrite dans J. Clin. Microbiol., 37(1), PP49-55, A. Troesch et al., 1999).

### Mode Opératoire :

Dans un tube, on mélange :
   - 18 µL de solution de marqueur à 250 mM (DMSO/méthanol 96/4), il s'agit soit du DCB soit du BBP,
   - 12 µL de DMSO,
   - 15 µL de tampon NASBA 0,5X (Kit "NucliSens Basic Kit Easy Q bioMérieux"),
   - 35 µL de tris HCL 1 M,
   - 5 µL de NASBA 0,1X (réaction d'amplification NASBA diluée dix fois, amplicon de 174 bases,
   - 15 µL de HCl 20 mM ou 15 µL d'eau, et
   - 15 µL d'eau afin de s'en servir comme témoin sans HCL.

La solution est mélangée par vortex puis incubée 10 min à 65°C.

### Purification des acides nucléiques :

Les acides nucléiques marqués ont été purifiés sur colonne QiaQuick (PCR purification kit QiaQuick, Qiagen, Hilden, Allemagne) en utilisant le protocole de purification recommandé par le fabricant. Le volume d'élution est de 100 µL.

### Hybridation sur puce à ADN :

Après purification, les acides nucléiques marqués sont transférés dans 400 µL de tampon d'hybridation. Les échantillons sont hybridés sur les puces à ADN conçues pour l'analyse de la séquence M20940 "GenBank" de l'ARN 16S de *Mycobacterium tuberculosis.* Cette puce à ADN est décrite par A. Troesch et al. publiée dans J. Clin. Microbiol., 37(1), PP49-55, 1999.

Les étapes d'hybridation ont été réalisées sur les stations fluidiques FS 450 (Affymetrix, Santa Clara, Californie, USA) en utilisant le protocole d'hybridation et les tampons décrits dans la publication précédente d'A. Troesch et al.

L'hybridation est révélée par le couplage de la streptavidine (SA) marquée à la phycoérythrine (PE), qui interagit avec la biotine des marqueurs utilisés dans les conditions suivantes : 300 µL d'eau pure ; 300 µL de tampon tris 100 mM pH 7 / NaCl 1 M / tween 20 0,05% / antimousse 0,005% ; 6 µL de BSA (50 mg/mL) ; 6 µL de SA-PE (300 µg/mL).

### Lecture de la puce à ADN :

La lecture de la fluorescence émise à la surface de la puce à ADN après marquage et hybridation ainsi que la génération des données en termes d'intensité du signal et de pourcentage d'homologie sont réalisés par les systèmes de lecture et le logiciel fournis par Affymetrix (Scanner Gene Chip Array et Logiciel GCOS) Le système de lecture fournit des intensités en signal et bruit de fond exprimées en rfu (« relative fluorescence unit », ou « unité de fluorescence relative »).

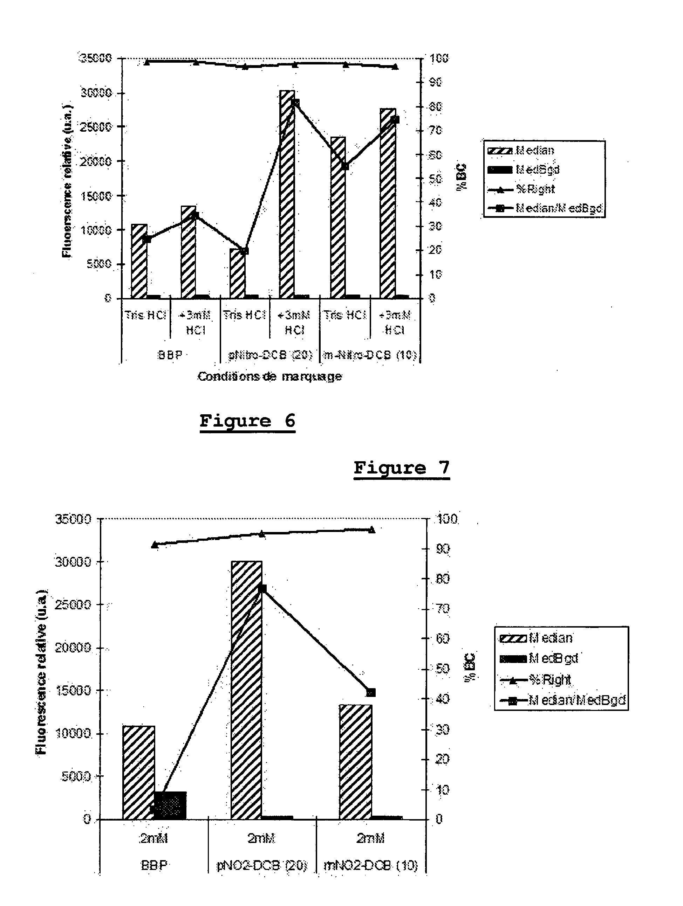

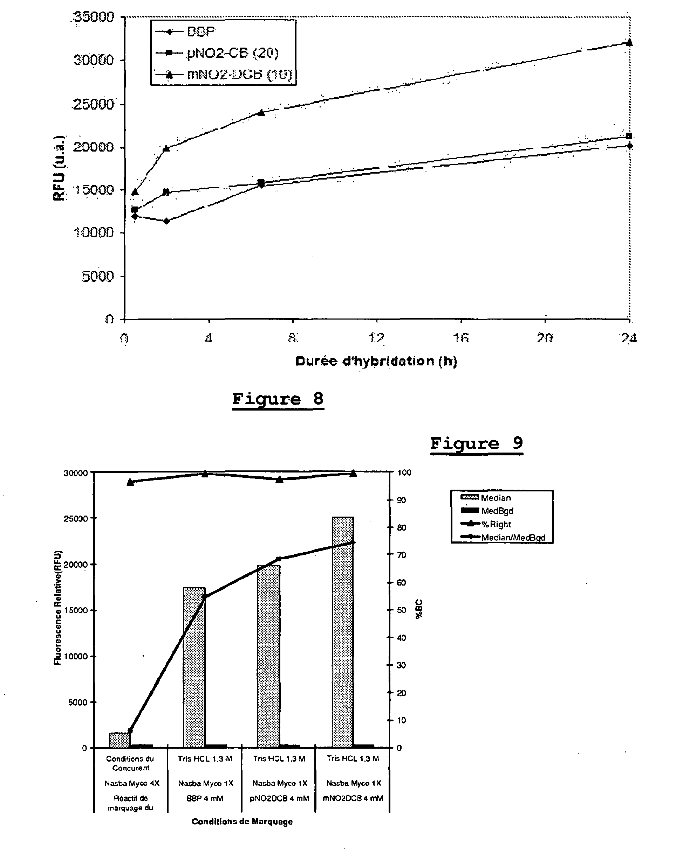

Le pourcentage d'homologie (%BC égal à %Right sur la figure 6 mais également ultérieurement sur les figures 7 et 9) est donné par rapport à une séquence de référence qui est dans ce cas la séquence de *Mycobacterium tuberculosis.*

Les résultats en termes d'intensité médiane du signal (Med), du bruit de fond (Med Bckgd) et du pourcentage d'homologie (%Right) sont donnés dans le graphique de la figure 6 pour les marqueurs BBP, *meta* Nitro DCB et *para* Nitro DCB.

### Résultats et conclusions :

D'une manière générale, c'est un pourcentage d'homologie supérieur à 90% qui est recherché en priorité. En second lieu, c'est un signal spécifique élevé et un bruit de fond faible qui sont souhaités.

Cet exemple montre qu'en absence d'acide chlorhydrique ajouté, le marqueur *meta* Nitro DCB présente les meilleurs résultats. En présence de 3 mM d'acide chlorhydrique ajoutés, les marqueurs *meta* Nitro DCB et *para* Nitro DCB présentent de meilleurs résultats que le marqueur BBP de référence.

Dans tous les cas et compte tenu de la très grande stabilité des DCB, ces derniers montrent leur supériorité par rapport aux molécules des précédentes générations, car leur capacité de marquage est au moins égale sinon supérieure à la molécule de référence BBP de deuxième génération.

### Exemple 6 : Marquage d'acides nucléiques par la meta ou para Nitro DCB par rapport à une molécule de BBP sans purification intermédiaire :

### Objectif :

On cherche à démontrer la même chose que dans l'exemple 5, mais en se plaçant dans des conditions défavorables, où l'excès de marqueur n'est pas purifié.

### Mode Opératoire :

Dans un tube de 1 mL, on mélange :
- 5 µL de solution de marqueur à 8 mM (DMSO/Méthanol 96/4). Il s'agit soit du DCB soit du BBP,
- 5 µL de NASBA 0.1 X (Kit NucliSens Basic Kit de bioMérieux),
- 5 µL de tris HCL 1M, et
- 5 µL de HCl 20 mM

La solution a été mélangée par vortex puis incubée 10 min à 65°C. Les acides nucléiques ainsi marqués ne sont pas purifiés, mais directement hybridés.

### Hybridation sur puce à ADN :

Les acides nucléiques marqués sont transférés sans purification dans 480 µL de tampon d'hybridation. Les échantillons sont hybridés sur les puces à ADN de la même façon que pour l'exemple précédent.

### Lecture de la puce à ADN :

Les résultats en termes d'intensité, de bruit de fond et de pourcentage d'homologie, pour les marqueurs BBP, *meta* Nitro DCB et *para* Nitro DCB, sont présentés dans le graphique de la figure 7.

### Résultats et conclusions :

Cet exemple montre que les marqueurs *meta* Nitro DCB et *para* Nitro DCB présentent, du fait de leur structure, des bruits de fond amoindris par rapport à la molécule de référence BBP. Les signaux spécifiques sont également améliorés (notamment pour *para* Nitro DCB), et le pourcentage d'homologie, relativement faible pour BBP, devient extrêmement important pour *meta* Nitro DCB.

Une fois encore, la supériorité des molécules de troisième génération par rapport à celles des précédentes générations est démontrée.

### Exemple 7 Evaluation de la stabilité sur 24h d'un amplicon marqué par le meta ou para Nitro DCB:

### Objectif :

On cherche à démontrer qu'un amplicon ARN marqué, peut être hybridé jusqu'à 24h sur une puce à ADN sans perdre de son intensité de fluorescence. Ce qui démontre la stabilité de la liaison ARN-marqueur.

### Mode Opératoire :

Dans un tube, on mélange :
- 5 µL de solution de marqueur (*meta* Nitro DCB ou *para* Nitro DCB) à 10 mM dans le DMSO/MeOH (96/4),
- 5 µL de produit d'amplification NASBA 0,1 X (Kit NucliSens Basic Kit de bioMérieux),
- 5 µL de Tris HCL 1 M, et
- 5 µL d'eau.

La solution est mélangée par vortex puis incubée 10 min à 65°C.

### Purification des acides nucléiques :

Les acides nucléiques marqués ont été purifiés sur colonne QiaQuick (PCR purification kit, Qiagen) en utilisant le protocole de purification recommandé par le fabricant. Le volume d'élution est de 100 µL.

### Hybridation sur puce à ADN :

Après purification, les acides nucléiques marqués sont transférés dans 400 µL de tampon d'hybridation. Les échantillons sont hybridés sur les puces à ADN conçues pour l'analyse de la séquence M20940 "GenBank" de l'ARN 16S de *Mycobacterium tuberculosis.* Cette puce à ADN est décrite dans la publication d'A. Troesch et al., J. Clin. Microbiol., 37(1), PP49-55, 1999.

Les étapes d'hybridation ont été réalisées en injectant 80 µL du mélange d'hybridation dans la puce puis en maintenant celle-ci dans un four à hybrider à 45°C pendant 0,5 heure, 2 heures, 6,5 heures ou 24 heures.

L'hybridation est révélée par le couplage de la streptavidine (SA) marquée à la phycoérythrine (PE), qui interagit avec la biotine des marqueurs utilisés dans les conditions suivantes : 300 pL d'eau pure ; 300 µL de tampon tris 100 mM pH 7 / NaCl 1 M / tween 20 0,05% / antimousse 0,005% ; 6 µL de BSA (50 mg/mL) ; 6 µL de SA-PE (300 µg/mL).

### Lecture de la puce à ADN :

La lecture de la fluorescence émise à la surface de la puce à ADN après marquage et hybridation ainsi que la génération des données en termes d'intensité du signal et du pourcentage d'homologie sont réalisés par les systèmes de lecture et le logiciel fournis par Affymetrix. Le système de lecture fournit des intensités en signal et bruit de fond exprimées en rfu ("relative fluorescence unit", ou "unité de fluorescence relative"). Le pourcentage d'homologie est donné par rapport à une séquence de référence qui est dans ce cas la séquence de *Mycobacterium tuberculosis.*

Les résultats en termes d'intensité médiane du signal (Med), en fonction de la durée d'hybridation sont donnés sur la figure 8 pour les marqueurs *meta* Nitro DCB (également appelé *m*-NO₂-DCB) et *para* Nitro DCB (également appelé *p*-NO₂-DCB).

Ils montrent que le signal de fluorescence reste stable et voire à tendance à monter en fonction de la durée de l'hybridation.

### Résultats et conclusions :

Cet exemple montre que les amplicons marqués avec le *meta* Nitro DCB ou *para* Nitro DCB restent parfaitement stable au cours de l'hybridation qui peut être prolongée pendant 24H (intérêt particulier pour les hybridations longues et expression des gènes en cancérologie), voir figure 8.

On observe même une augmentation du signal de fluorescence au cours du temps qui est due à une meilleure hybridation des amplicons (la cinétique d'hybridation est lente).

On démontre ainsi la stabilité de la liaison marqueur-acide nucléique.

### Exemple 8 Comparaison de l'efficacité de marquage des molécules décrites dans la présente invention celle d'une technologie commercialement disponible (ULS RNA Labelling Kit, Kreatech, Pays-Bas) :

### Mode Opératoire :

Les amplicons ARN sont préparés par une amplification NASBA comme précédemment et sont marqués avec les molécules BBP, p-NO₂-DCB ou m-NO₂-DCB.

Dans un tube, on mélange :
- 5 µL de NASBA 1X (Kit NucliSens Basic Kit de bioMérieux),
- 5 µL de solution de marqueur à 20 mM (BBP, *p*-NO₂-DCB ou *m-*NO₂-DCB dans le DMSO/Métanol 96/4,
- 5 µL de Tris HCL 1 M pH 7,4 et
- 5 µL d'eau.

La solution a été mélangée par vortex puis incubée 10 min à 65°C.

Pour le marquage avec le kit commercial de Kreatech, le protocole recommandé par le fournisseur a été suivi. En résumé ; on mélange :
- 20 µL de NASBA 1X (Kit NucliSens Basic Kit de bioMérieux),
- 1 µL de solution de marqueur,
- 3 µL Tampon 10x, et
- 6 µL d'eau.

La solution a été incubée 30 min à 85°C

### Purification des acides nucléiques :

Les acides nucléiques marqués à l'aide des molécules BBP, *p-*NO₂-DCB ou *m*-NO₂-DCB ont été purifiés sur colonne QiaQuick (PCR purification kit, Qiagen) en utilisant le protocole de purification recommandé par le fabricant. Le volume d'élution est de 100 µL.

Pour les acides nucléiques marqués à l'aide du kit commercial, la purification recommandée et fournie par Kreatech a été utilisée. Le volume final est de 30 µL, auxquels on ajoute 100 µL d'une solution de blocage recommandée par cette société.

### Hybridation sur puce à ADN :

Après purification, les acides nucléiques marqués sont transférés dans 400 µL de tampon d'hybridation (BBP, *p*-NO₂-DCB ou *m*-NO₂-DCB) ou 370 µL de tampon d'hybridation (Kreatech).

Lesdits acides nucléiques sont hybridés sur les puces à ADN conçues pour l'analyse de la séquence M20940 « GenBank » de l'ARN 16S de *Mycobacterium tuberculosis.*

Cette puce à ADN est décrite dans la publication de A. Troesch et al., J. Clin. Microbiol., 37(1), PP49-55, 1999. Les étapes d'hybridation ont été réalisées sur les stations fluidiques (Affymetrix FS 450) en utilisant le protocole d'hybridation et les tampons décrits dans cette publication de A. Troesch *et al.*

L'hybridation est révélée par le couplage de la streptavidine (SA) marquée à la phycoérythrine (PE), qui interagit avec la biotine des marqueurs utilisés dans les conditions suivantes : 300 µL d'eau pure ; 300 µL de tampon tris 100 mM pH 7 / NaCl 1 M / tween 20 à 0,05% / antimousse 0,005% ; 6 µL de BSA (50 mg/mL) ; 6 µL de SA-PE (300 µg/mL).

### Lecture de la puce à ADN :

La lecture de la fluorecence émise à la surface de la puce à ADN après marquage et hybridation ainsi que la génération des données en termes d'intensité du signal et de pourcentage d'homologie sont réalisés par les systèmes de lecture et le logiciel fourni par Affymetrix (Gen Chip array et logiciel GCOS). Le système de lecture fournit des intensités en signal et bruit de fond exprimées en rfu ("relative fluorescence unit", ou "unité de fluorescence relative"). Le pourcentage d'homologie est donné par rapport à une séquence de référence qui est dans ce cas la séquence de *Mycobacterium tuberculosis.*

Les résultats en termes d'intensité médiane du signal (Med), du bruit de fond (Med Bckgd) et du pourcentage d'homologie (%BC) sont donnés dans la figure 9 pour les marqueurs BBP, *m*-NO₂-DCB et *p*-NO₂-DCB ainsi que pour le kit concurrent.

### Résultats et conclusions :

On constate que la technologie utilisant les marqueurs cis-platine de Kreatech (appliquée exactement dans les conditions décrites par le fournisseur) à un potentiel de marquage bien inférieur à la solution technique apportée par la présente invention, puisqu'il faut rajouter plus de quatre fois la concentration d'ARN pour avoir un signal nettement détaché du bruit de fond chez Kreatech (Figure 9), mais qui reste dans tous les cas plus de dix fois plus faible que le marquage réalisé avec les molécules DCB ou BBP.

Dans tous les cas le pourcentage d'identification (%BC) reste le même.

Le marquage sur les liens internucléosidiques en comparaison à une autre technique de marquage permet donc d'obtenir une sensibilité de détection bien meilleure et cela quelles que soient les générations de molécules.

## Revendications

1. Réactif de marquage de formule (A) 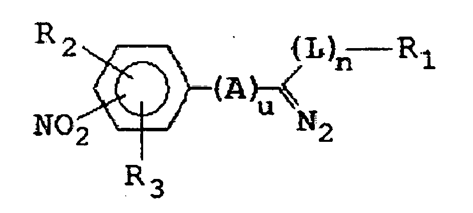 dans laquelle :
• R₁ représente un marqueur détectable ou au moins deux marqueurs détectables reliés entre eux par au moins une structure multimérique,
• R₂ et R₃ représentent indépendamment l'un de l'autre : H, NO₂, Cl, Br, F, I, R₄-(L)ₙ-Y-X-, OR, SR, NR², R, NHCOR, CONHR, COOR avec R = alkyle ou aryle et R₄ représente un marqueur détectable ou au moins deux marqueurs détectables reliés entre eux par au moins une structure multimérique,
• L est un bras de liaison comportant un enchaînement linéaire d'au moins deux liaisons covalentes,
• n est un nombre entier égal à 1,
• A est un bras de liaison comportant au moins une double liaison covalente permettant la conjugaison de la fonction diazo avec le cycle aromatique,
• u est un nombre entier compris entre 0 et 2, et
• -Y-X- représente -CONH-, -NHCO-, -CH₂O-, -CH₂S-.

2. Réactif de marquage, selon la revendication 1, de formule (C) : 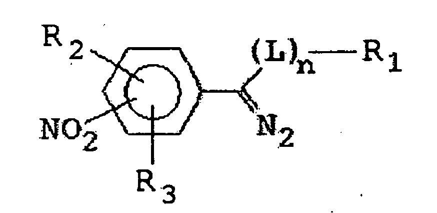 dans laquelle :
• R₁ représente un marqueur détectable ou au moins deux marqueurs détectables reliés entre eux par au moins une structure multimérique,
• R₂ et R₃ représentent indépendamment l'un de l'autre : H, NO₂, Cl, Br, F, I, R₄-(L)ₙ-Y-X-, OR, SR, NR², R, NHCOR, CONHR, COOR avec R = alkyle ou aryle, et R₄ représente un marqueur détectable ou au moins deux marqueurs détectables reliés entre eux par au moins une structure multimérique,
• L est un bras de liaison comportant un enchaînement linéaire d'au moins deux liaisons covalentes,
• n est un nombre entier égal 1, et
• -Y-X- représente -CONH-, -NHCO-, -CH₂O-, -CH₂S-.

3. Réactif de marquage, selon l'une quelconque des revendications 1 ou 2, de formule (E):

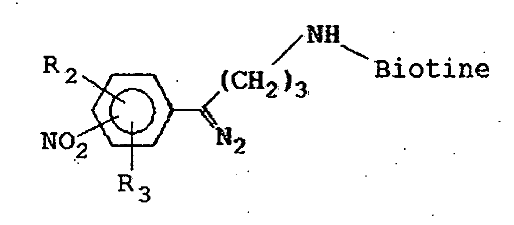

4. Réactif, selon l'une quelconque des revendications 1 à 3, **caractérisé par le fait que** le groupement nitro est en position *meta* ou *para.*

5. Réactif, selon l'une quelconque des revendications 1 à 4, **caractérisé par le fait que** R₁ est constitué par un résidu D-Biotine de formule (F) :

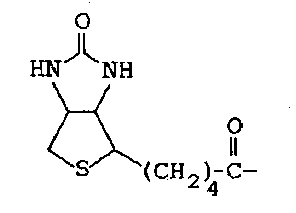

6. Procédé de synthèse d'un réactif de marquage, selon l'une quelconque des revendications 1 à 5, comprenant les étapes suivantes :
a) un dérivé d'acide carboxylique est mis en réaction avec l'énolate d'une lactone (réaction de type Claisen) pour former un précurseur cyclique,
b) lequel précurseur cyclique est ensuite ouvert avec un acide halogéné pour former une cétone aromatique halogénée,
c) la fonction carbonyle de la cétone aromatique halogénée est protégée par un groupement protecteur pour former un précurseur protégé,
d) lequel précurseur protégé est soumis à une réaction d'amination (de type Gabriel) pour former un précurseur aminé,
e) lequel précurseur aminé est déprotégé pour libérer la fonction amine, ladite fonction amine étant mise en réaction avec un marqueur détectable dont la fonction carboxylique est activée pour former un précurseur comportant un marqueur détectable,
f) le précurseur marqué est soumis à une réaction de déprotection de la fonction carbonyle pour former un précurseur marqué et carbonylé, enfin
g) le précurseur marqué et carbonylé est transformé en un réactif de marquage, selon l'une quelconque des revendications 1 à 5 par une transformation de la fonction carbonyle en une fonction diazo (du type Bamford Stevens).

7. Procédé pour le marquage d'une molécule biologique, comprenant la mise en contact en solution homogène, dans un tampon sensiblement aqueux, d'une molécule biologique et d'un réactif, selon l'une quelconque des revendications 1 à 5.

8. Molécule biologique marquée par le procédé, selon la revendication 7.

9. Procédé de marquage et de fragmentation d'un acide nucléique simple ou double brin comprenant les étapes suivantes :
• fragmenter l'acide nucléique,
• attacher un marqueur sur au moins un des fragments par l'intermédiaire d'un réactif de marquage choisi parmi les réactifs, selon l'une quelconque des revendications 1 à 5,
ledit réactif se couplant de manière covalente et majoritaire sur au moins un phosphate dudit fragment.

10. Procédé, selon la revendication 9, **caractérisé par le fait que** la fragmentation et le marquage sont effectués en deux étapes.

11. Procédé, selon la revendication 9, **caractérisé par le fait que** la fragmentation et le marquage sont effectués en une étape.

12. Procédé, selon l'un quelconque des revendications 9 à 11, **caractérisé par le fait que** le marquage s'effectue en solution homogène sensiblement aqueuse.

13. Procédé, selon l'une quelconque des revendications 9 à 11, **caractérisé par le fait que** la fragmentation s'effectue par voie enzymatique, physique ou chimique.

14. Acide nucléique marqué par le procédé, selon l'une quelconque des revendications 9 à 13.

15. Kit de détection d'un acide nucléique cible comprenant un acide nucléique marqué, selon la revendication 14.

16. Support solide sur lequel est fixé un réactif, selon l'une quelconque des revendications 1 à 5.

17. Procédé de capture d'acides nucléiques comprenant les étapes suivantes :
• on dispose d'un support solide sur lequel est fixé directement ou indirectement au moins une molécule biologique, selon la revendication 8 ou un acide nucléique, selon la revendication 14, la molécule biologique ou l'acide nucléique comprenant une fonction diazométhyle,
• on met en contact un échantillon biologique susceptible de contenir des acides nucléiques libres, et
• on lave le support solide où la (ou les) molécule(s) est/sont fixée(s) de manière covalente au moins à un acide nucléique.

## Patentansprüche

1. Ein Markierungsreagens mit der Formel (A) 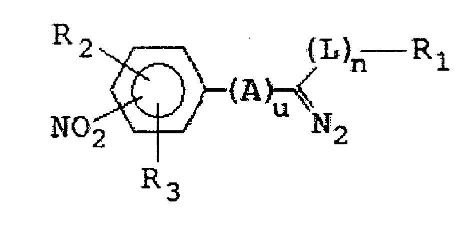 wobei:
• R₁ einen nachweisbaren Marker oder mindestens zwei nachweisbare Marker, die miteinander durch mindestens eine multimere Struktur verbunden sind, darstellt,
• R₂ und R₃ unabhängig voneinander Folgende darstellen: H, NO₂, Cl, Br, F, I, R₄-(L)ₙ-Y-X-, OR, SR, NR², R, NHCOR, CONHR, COOR, wobei R = Alkyl oder Aryl ist, und R₄ einen nachweisbaren Marker oder mindestens zwei nachweisbare Marker, die miteinander durch mindestens eine multimere Struktur verbunden sind, darstellt,
• L ein Bindungsarm ist, der eine lineare Verknüpfung von mindestens zwei kovalenten Bindungen beinhaltet,
• n eine ganze Zahl gleich 1 ist,
• A ein Bindungsarm ist, der mindestens eine kovalente Doppelbindung, die die Konjugation der Diazofunktion mit dem aromatischen Ring ermöglicht, beinhaltet,
• u eine ganze Zahl zwischen 0 und 2 ist und
• Y-X- -CONH-, -NHCO-, -CH₂O-, -CH₂S- darstellt.

2. Markierungsreagens gemäß Anspruch 1 mit der Formel (C): 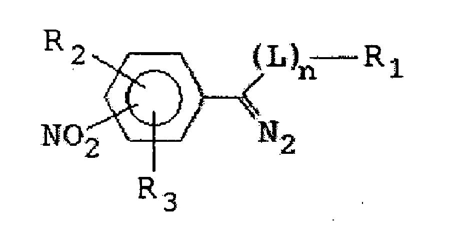 wobei:
• R₁ einen nachweisbaren Marker oder mindestens zwei nachweisbare Marker, die miteinander durch mindestens eine multimeren Struktur verbunden sind, darstellt,
• R₂ und R₃ unabhängig voneinander Folgende darstellen: H, NO₂, Cl, Br, F, I, R₄-(L)ₙ-Y-X-, OR, SR, NR², R, NHCOR, CONHR, COOR, wobei R = Alkyl oder Aryl ist, und R₄ einen nachweisbaren Marker oder mindestens zwei nachweisbare Marker, die miteinander durch mindestens eine multimere Struktur verbunden sind, darstellt,
• L ein Bindungsarm ist, der eine lineare Verknüpfung von mindestens zwei kovalenten Bindungen beinhaltet,
• n eine ganze Zahl gleich 1 ist und
• Y-X- -CONH-, -NHCO-, -CH₂O-, -CH₂S- darstellt.

3. Markierungsreagens gemäß einem der Ansprüche 1 oder 2 mit der Formel (E):

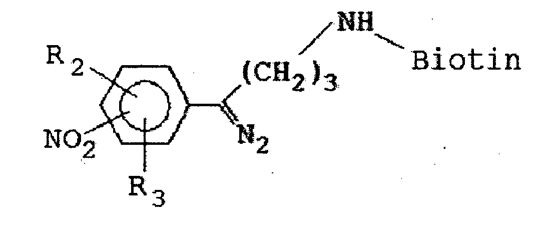

4. Reagens gemäß einem der Ansprüche 1 bis 3, **dadurch gekennzeichnet, dass** die Nitrogruppe in der Meta-oder Para-Position ist.

5. Reagens gemäß einem der Ansprüche 1 bis 4, **dadurch gekennzeichnet, dass** R₁ aus einem D-Biotin-Rest der nachfolgenden Formel (F) besteht:

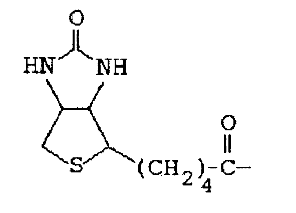

6. Ein Verfahren zur Synthese eines Markierungsreagens gemäß einem der Ansprüche 1 bis 5, umfassend die folgenden Schritte:
a) ein Carbonsäure-Derivat wird mit dem Enolat eines Lactons (der Art Claisen-Reaktion) eine Reaktion eingehen gelassen, um einen zyklischen Vorläufer zu bilden,
b) der zyklische Vorläufer wird dann mit einer Halogensäure geöffnet, um ein halogeniertes aromatisches Keton zu bilden,
c) die Carbonylfunktion des halogenierten aromatischen Ketons wird durch eine Schutzgruppe geschützt, um einen geschützten Vorläufer zu bilden,
d) der geschützte Vorläufer wird einer Aminierungsreaktion (der Art Gabriel) unterzogen, um einen aminierten Vorläufer zu bilden,
e) der aminierte Vorläufer wird entschützt, um die Aminfunktion freizugeben, wobei die Aminfunktion mit einem nachweisbaren Marker, dessen Carboxylfunktion aktiviert ist, zur Reaktion gebracht wird, um einen Vorläufer, der einen nachweisbaren Marker beinhaltet, zu bilden,
f) der markierte Vorläufer wird einer Carbonylfunktion-Entschützungsreaktion unterzogen, um einen markierten und carbonylierten Vorläufer zu bilden, und schließlich
g) der markierte und carbonylierte Vorläufer wird in ein Markierungsreagens umgesetzt, gemäß einem der Ansprüche 1 bis 5, durch eine Umsetzung der Carbonylfunktion in eine Diazofunktion (der Art Bamford-Stevens).

7. Ein Verfahren zur Markierung eines biologischen Moleküls, umfassend das In-Kontakt-Bringen in einer homogenen Lösung, in einem im Wesentlichen wässrigen Puffer, eines biologischen Moleküls und eines Reagens gemäß einem der Ansprüche 1 bis 5.

8. Ein biologisches Molekül, das durch das Verfahren gemäß Anspruch 7 markiert ist.

9. Ein Verfahren zur Markierung und Fragmentierung einer ein- oder doppelsträngigen Nukleinsäure, umfassend die folgenden Schritte:
• Fragmentieren der Nukleinsäure,
• Anbringen eines Markers an mindestens einem der Fragmente über ein Markierungsreagens, das ausgewählt ist aus den Reagenzien gemäß einem der Ansprüche 1 bis 5,
wobei sich das Reagens auf kovalente und überwiegende Weise an mindestens ein Phosphat des Fragments koppelt.

10. Verfahren gemäß Anspruch 9, **dadurch gekennzeichnet, dass** die Fragmentierung und die Markierung in zwei Schritten durchgeführt werden.

11. Verfahren gemäß Anspruch 9, **dadurch gekennzeichnet, dass** die Fragmentierung und die Markierung in einem Schritt durchgeführt werden.

12. Verfahren gemäß einem der Ansprüche 9 bis 11, **dadurch gekennzeichnet, dass** die Markierung in einer im Wesentlichen wässrigen homogenen Lösung durchgeführt wird.

13. Verfahren gemäß einem der Ansprüche 9 bis 11, **dadurch gekennzeichnet, dass** die Fragmentierung enzymatisch, physikalisch oder chemisch durchgeführt wird.

14. Eine Nukleinsäure, die durch das Verfahren gemäß einem der Ansprüche 9 bis 13 markiert ist.

15. Ein Kit zum Nachweis einer Ziel-Nukleinsäure, umfassend eine markierte Nukleinsäure gemäß Anspruch 14.

16. Ein fester Träger, an den ein Reagens gemäß einem der Ansprüche 1 bis 5 gebunden ist.

17. Ein Verfahren zur Erfassung von Nukleinsäuren, umfassend die folgenden Schritte:
• Bereitstellen eines festen Trägers, an den mindestens ein biologisches Molekül gemäß Anspruch 8 oder eine Nukleinsäure gemäß Anspruch 14 direkt oder indirekt gebunden ist, wobei das biologische Molekül oder die Nukleinsäure eine Diazomethylfunktion umfassen,
• In-Kontakt-Bringen einer biologischen Probe, die freie Nukleinsäuren enthalten kann, und
• Waschen des festen Trägers, wo das Molekül (oder die Moleküle) kovalent an mindestens eine Nukleinsäure gebunden ist (sind).

## Claims

1. A labelling reagent of formula (A) 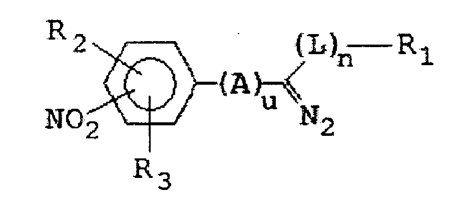 wherein:
• R₁ represents a detectable label or at least two detectable labels linked to one another by at least one multimeric structure,
• R₂ and R₃ represent independently of one another: H, NO₂, Cl, Br, F, I, R₄-(L)ₙ-Y-X-, OR, SR, NR², R, NHCOR, CONHR, COOR, where R = alkyl or aryl and R₄ represents a detectable label or at least two detectable labels linked to one another by at least one multimeric structure,
• L is a linker arm comprising a linear chain of at least two covalent bonds,
• n is an integer equal to 1,
• A is a linker arm comprising at least one covalent double bond which makes it possible to conjugate the diazo function with the aromatic ring,
• u is an integer between 0 and 2, and
• -Y-X- represents -CONH-, -NHCO-, -CH₂O-, -CH₂S-.

2. The labelling reagent, according to claim 1, of formula (C): 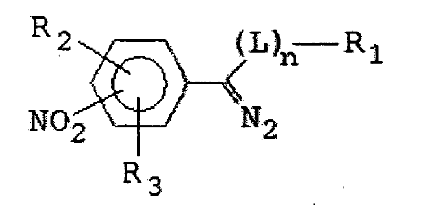 wherein:
• R₁ represents a detectable label or at least two detectable labels linked to one another by at least one multimeric structure,
• R₂ and R₃ represent independently of one another: H, NO₂, Cl, Br, F, I, R₄-(L)ₙ-Y-X-, OR, SR, NR², R, NHCOR, CONHR, COOR, where R = alkyl or aryl, and R₄ represents a detectable label or at least two detectable labels linked to one another by at least one multimeric structure,
• L is a linker arm comprising a linear chain of at least two covalent bonds,
• n is an integer equal to 1, and
• -Y-X- represents -CONH-, -NHCO-, -CH₂O-, -CH₂S-.

3. The labelling reagent, according to any one of claims 1 or 2, of formula (E):

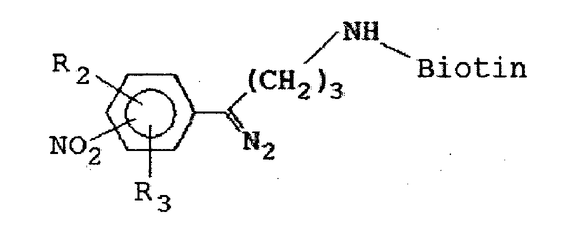

4. The reagent, according to any one of claims 1 to 3, **characterised by** the fact that the nitro group is in *meta* or *para* position.

5. The reagent, according to any one of claims 1 to 4, **characterised by** the fact that R₁ is made up of a D-biotin residue of formula (F):

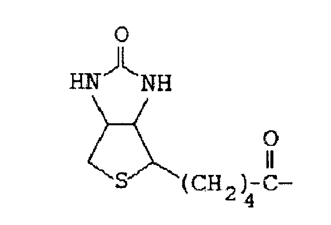

6. A method of synthesising a labelling reagent according to one of claims 1 to 5, comprising the following steps:
a) a carboxylic acid derivative is reacted with the enolate of a lactone (reaction of the Claisen type) to form a cyclic precursor,
b) said cyclic precursor is then opened with a halogen acid to form a halogenated aromatic ketone,
c) the carbonyl function of the halogenated aromatic ketone is protected by a protective group to form a protected precursor,
d) said protected precursor is subjected to an amination reaction (of the Gabriel type) to form an aminated precursor,
e) said aminated precursor is deprotected to release the amine function, said amine function being reacted with a detectable label whose carboxyl function is activated to form a precursor including a detectable label,
f) the labelled precursor is subjected to a carbonyl function deprotection reaction to form a labelled and carbonylated precursor, and finally
g) the labelled and carbonylated precursor is converted into a labelling reagent, according to any one of claims 1 to 5 by conversion of the carbonyl function into a diazo function (of the Bamford Stevens type).

7. A method for labelling a biological molecule, comprising contacting in homogeneous solution, in a substantially aqueous buffer, a biological molecule and a reagent, according to any one of claims 1 to 5.

8. A biological molecule labelled by the method, according to claim 7.

9. A method of labelling and fragmenting a single-stranded or double-stranded nucleic acid, comprising the following steps:
• fragmenting the nucleic acid,
• attaching a label onto at least one of the fragments via a labelling reagent selected from the reagents according to any one of claims 1 to 5,
said reagent coupling covalently and predominantly onto at least one phosphate of said fragment.

10. The method according to claim 9, **characterised by** the fact that the fragmenting and labelling are performed in two steps.

11. The method according to claim 9, **characterised by** the fact that the fragmenting and labelling are performed in one step.

12. The method according to any one of claims 9 to 11, **characterised by** the fact that labelling is performed in a substantially aqueous homogeneous solution.

13. The method according to any one of claims 9 to 11, **characterised by** the fact that the fragmenting is performed enzymatically, physically or chemically.

14. A nucleic acid labelled by the method, according to any one of claims 9 to 13.

15. A kit for detecting a target nucleic acid comprising a labelled nucleic acid, according to Claim 14.

16. A solid support on which is bound a reagent according to any one of claims 1 to 5.

17. A method of capturing nucleic acids, comprising the following steps:
• providing a solid support on which is bound, directly or indirectly, at least one biological molecule according to claim 8 or a nucleic acid, according to claim 14, the biological molecule or nucleic acid comprising a diazomethyl function,
• placing in contact a biological sample which may contain free nucleic acids, and
• washing the solid support where the molecule(s) is/are bound covalently at least to one nucleic acid.
